# EUROPEAN PATENT SPECIFICATION

(11) **EP 0 654 988 B1**
(45) Date of publication and mention of the grant of the patent: **04.03.1998**
(21) Application number: 94919438.5
(22) Date of filing: 09.06.1994
(51) Int. Cl.: A61J 1/05, A61M 39/00

(54) **IMPROVED PORT PROTECTOR AND CONTAINERS HAVING SAME**
VERBESSERTER SCHUTZ FÜR ÖFFNUNG SOWIE DAMIT VERSEHENER BEHÄLTER
DISPOSITIF DE PROTECTION AMELIORE POUR ORIFICE D'ACCES ET RECIPIENTS LE COMPRENANT

(30) Priority: 10.06.1993 US 75158
(43) Date of publication of application: 31.05.1995
(73) Proprietor: BAXTER INTERNATIONAL INC., Deerfield, Illinois 60015 (US)
(72) Inventor: BALTEAU, Patrick, B-5100 Jambres (BE); EERLINGEN, V., B-3010 Leuven (BE); LO, Ying-Cheng, Green Oaks, Il 60048 (US); WOO, Lecon, Livertyville, IL 60048 (US); CHAN, Eddie, Mundelein, IL 60060 (US); LAURIN, Dean, Hawthorn Wood, IL 60047 (US); LING, Michael, T., K., Vernon Hills, IL 60061 (US); PELUSO, Francesco, B-3001 Heverlee (BE)
(74) Representative: MacGregor, Gordon
(86) International application number: US9406537
(87) International publication number: WO9428855

(56) References cited:
- WO-A-90/06143
- US-A- 4 335 756
- US-A- 4 573 980
- US-A- 4 616 760

## Description

### BACKGROUND OF THE INVENTION

The present invention relates generally to removable devices for maintaining a sterile environment within the interior of a tubular member such as a port prior to use

The preamble of claim 1 is based on US-A-4 573 980.

It is known to house medical solutions and products in flexible plastic containers. These containers provide a means for housing the solution prior to the solution being administered to a patient or used for other therapeutic applications.

In order to access the interior of the container, either to infuse solution therein or access a solution contained therein, it is known to provide the containers with one or more ports or fitments. These ports are typically tubular in shape and define a flow path from an interior of the container to the outside environment.

Such ports may include a pierceable membrane or injection site. In use, fluid is either added to the container or accessed therefrom by inserting a needle, cannula, or other member through the port piercing the membrane or injection site.

It is also known to connect a tubing to the port to provide a fluid path to and/or away from the container. The tubing can terminate in a cannula or luer connector to allow the tubing to be coupled to another container or a patient.

In order to prevent contamination of the solution, and infection to the patient, the transfer of solution out of the container, and in many instances into the container, must take place under sterile conditions. Therefore, the distal ends of the port or connector (e.g., cannula, luer connector, etc.) are frequently capped with a "port protector." The function of the port protector is to preserve the sterile integrity of the interior of the port or connector after the entire container assembly has been assembled and terminally sterilized. Most frequently, sterilization in the medical industry is through the use of steam sterilization. Steam sterilization typically takes place under elevated temperatures and pressure, such as, for example, 121°C (250°F).

Once sterilized, the port protector must prevent any microbial ingress from the exterior, non-sterile environment, from reaching the interior of the port or connector. Thus, a tight seal at the interface is one of the most important functions of the port protector.

One of the disadvantages of a tight seal, however, is that it also prevents steam, during the steam sterilization process, from reaching the interior of the port or connector. This is particularly true of the overlapping surfaces that must be sterilized in order to insure a total kill of microbial burdens present prior to steam sterilization.

Currently, one method used to provide the necessary requirements of a port protector is to use a plastic sleeve, typically constructed from extruded flexible polyvinyl chloride, that is sealed off at one end with a slit opening cut along the longitudinal direction of the sleeve. During assembly, the sleeve is spread open at the slit and slipped onto the tube through the pre-slit opening. The sealed end is placed against the opening of the tube to provide the protective function. During steam sterilization, steam can penetrate through the sleeve so that a sufficient microbial kill is achieved. During use, ideally, the user or patient grabs the slit end of the sleeve and pulls it away from the tube to expose the tube for use.

Although these pre-slit sleeves have been in use for many years, there are a number of issues with respect to the use of same. For example, frequently, the sleeve develops a strong tack bond with the tube during steam sterilization. This can make it very difficult to remove.

Additionally, the thin walled slit part of the sleeve often becomes severely distorted during steam sterilization. This can make it extremely difficult for a patient with visual or manual impairment to remove the sleeve.

Still further, the dimensions of the flexible sleeve are difficult to control. Accordingly, relaxations of the material may occur during the steam sterilization process, causing the sleeve to come off at a later time, thereby breaching the sterile barrier.

Furthermore, during the extrusion manufacturing of the sleeve, die lines are frequently introduced in the longitudinal direction. This can reduce the sterile barrier, or at times, render the microbial barrier ineffective.

Additionally, sometimes in systems with closed ends, pressure differentials are created during sterilization. These pressure differentials can blow off the sleeve, destroying the sterile barrier.

One approach that has been used in an attempt to overcome the disadvantages of the sleeve is to use an injection molded cap to insure the sterility of the surfaces under the protector cap. An example of such a cap is set forth in U.S. Patent No. 4,573,980.

One of the disadvantages of the structure is that steam cannot reach the interior of the connector or tube when it is closed off by the cap. Therefore, it is necessary to sterilize the connector and cap with an ionizing radiation and then assemble the assembly to a container. After this step, it is then necessary to terminally sterilize the entire container assembly using steam.

This practice, while accomplishing the stated sterility objectives adds significant burden to the materials of construction. For example, the entire cap/tube sub-assembly is required to withstand both sterilizing doses of radiation, and subsequently withstand the elevated temperatures and pressure in a steam autoclave.

Furthermore, it has been found that many material degradation products are created during the radiation sterilization. A number of these products are easily hydrolyzed and become water soluble during steam sterilization. This therefore increases the leachable contaminants that can reach the patient.

Another consequence of the requirement of double sterilization is the added cost associated with the radiation sterilization step. Still further, the additional necessary transportation, segregation, and inventory of sterile and non-sterile assemblies could, arguably, result in a mix-up of sterile and non-sterile products that could have serious consequences to a patient's safety.

Still further, with respect to certain medical procedures that require the transfer of fluids to and from a container, such as continuous ambulatory peritoneal dialysis (CAPD), due to the construction of typical port protectors, it is necessary for other components to be present in the container assembly. In an embodiment, CAPD requires the use of twin containers. One container includes the solution to be infused into the patient and the other container functions as the drain bag. A tube provides a fluid path from the solution container to a luer connector. Another fluid path is provided from the luer connector to the drain bag. This allows patients to drain their full peritoneal cavity into the drain bag prior to infusing fresh dialysis solution, using the luer connector.

A port protector, such as those discussed above, is typically located over the luer connector. Due to the structure of the port protector, aside from the sterilization issues set forth above, another issue is raised. It is necessary to place in the drain line a frangible cannula to avoid air evacuation and subsequent tube collapse during sterilization. It would be desirable from a manufacturing and consumer standpoint to remove the frangible cannula from the drain line.

There is therefore a need for an improved port protector and/or container assembly.

### SUMMARY OF THE INVENTION

The present invention provides an improved port protector and container assembly having same. The port protector eliminates the need for a double sterilization process, while allowing a terminal sterilization of the capped port or connector. Additionally, the port protector of the present invention fulfills all of the functional requirements necessary for a port protector. Still further, the present invention minimizes the potential degradation products from the ionizing radiation from reaching the patient by eliminating the radiation step entirely. The port protector, and container assembly using same, is more cost effective as compared to prior assemblies.

US-A-4,335,756 discloses a port protector which, when fitted to the end of a tubular member, permits a sterilizing agent, such as ethylene oxide gas, to pass between the protector and the tubular member, thereby sterilizing the interior of the tube. US-A-4,616,760 discloses a steam permeable cap for a sterile solution container.

The present invention provides A port protector removably attached over an open end of a tubular member, the port protector comprising:
a cap portion including a planar closure and side walls defining an interior receiving said open end and sealingly engaging the tubular member, the cap portion at least being constructed from a thermoplastic elastomer; a ring extending from the cap portion to facilitate removal of the cap portion from the tubular member, the ring being attached directly to the side walls and extending from opposite points on the side walls forming an enclosed opening between the ring and the cap, the ring extending from the end of the side walls opposite to said open end,
   characterised in that the closure defines a top of the cap portion, extending over said open end and spaced from the ring by the side walls, the ring extends in a plane in parallel spaced relation to the plane of said closure, and the closure (18) has a sufficiently thin cross-sectional thickness to allow steam to pass through the top into the opening of the tubular member during steam sterilization.

In an embodiment of the port protector, the top of the cap has a thickness of equal to or less than 0.65 millimeters. The entire port protector may be constructed from the same material and the port protector may include touch indicators for providing means for identifying products through touch. At least portions of the port protector may be color coded to provide visual means for identifying product.

In an embodiment, the port protector is constructed from a material chosen from the group consisting of: thermoplastic olefinic elastomers; styrene thermoplastic, elastomers; polyamide thermoplastic elastomers; and combinations of same wherein the composition includes at least 23% weight percent hydrophilic polymeric components.

The port protector described herein does not require the use of lubricants to position the port protector on a connector or port.

Moreover, the described port protector can be used on a CAPD assembly without the need for a frangible in the drain line.

The described port protector may easily be automatically assembled to the port it is intended for and may easily be removed, even by weak or disabled patients.

Additional features and advantages of the present invention are described in, and will be apparent from, the detailed description of the presently preferred embodiments and from the drawings.

### BRIEF DESCRIPTION OF THE DRAWINGS

Figure 1 illustrates a perspective view of the port protector of the present invention.

Figure 2 illustrates a cross-sectional view of the port protector of the present invention taken along lines II-II of Figure 1.

Figure 3 illustrates an embodiment of the port protector used on a CAPD container assembly.

### DETAILED DESCRIPTION OF THE PRESENTLY PREFERRED EMBODIMENTS

The present invention provides an improved port protector. As used herein, "port protector" refers to a device for maintaining a sterile barrier at an opening of a tubular member, such as a port or other connector, that provides a fluid path from an interior to a second environment. Accordingly, the port protector of the present invention can be used on a wide variety of tubular members including ports or fitments located on containers as well as, for example, luer connectors at the end of a fluid line.

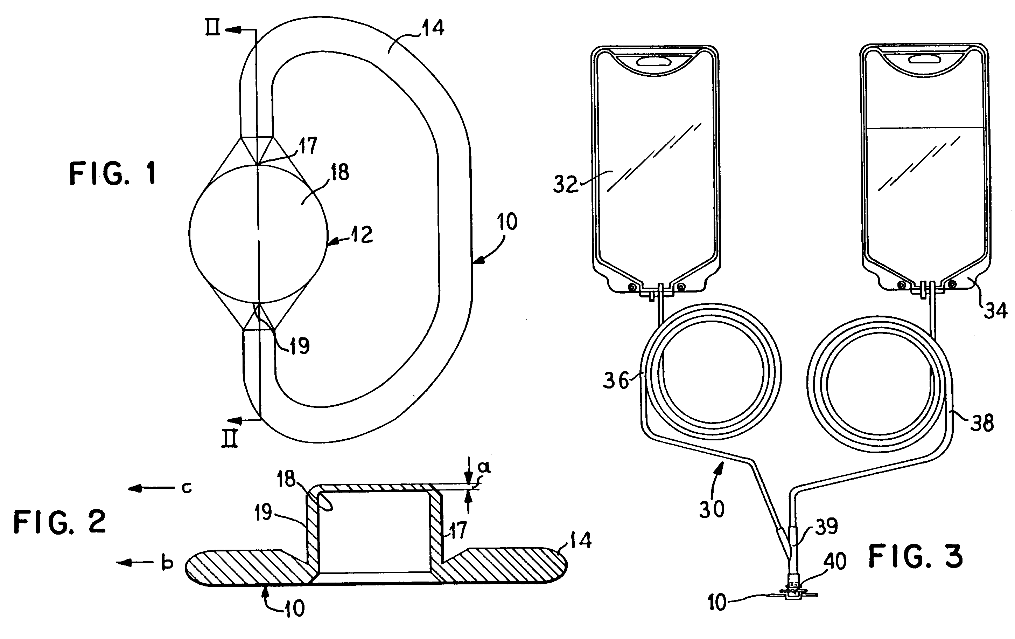

Referring now to Figures 1 and 2, an embodiment of the port protector is illustrated. As illustrated, the port protector 10 includes a cap 12 and a pull ring 14. The cap 12 includes side walls 17 and 19 and a top 18. At least the top 18 of the port protector is sufficiently thin and of adequate composition to allow steam to penetrate the top and enter an interior of a connector such as that illustrated in Figure 3.

In this regard, preferably, the top 18 has a thickness "a" that is less than or equal to 0.65 millimeters. It has been found that with such a thickness, when the cap 12 is constructed from a thermoplastic elastomer, the cap will allow steam to penetrate the top 18 of the cap and enter an interior of a tubular member to which the port protector 10 is secured.

The pull ring 14 extends from opposite sides 17 and 19 of the top 18, wherein a plane "b" defined by the pull ring 14 is parallel to a plane "c" defined by a top 18 of the cap 12. Therefore, a simplified mold can be used that only includes two mating halves.

The inner surface of the cap 12 is preferably smooth. This insures a good seal on the port or connector over which the port protector 10 is located.

If desired, means can be provided on the port protector 10 for allowing a patient or other user to identify the contents. This means can include color coding the port protector 10 so that the color will indicate the contents of the container. If the product is to be used by a visually impaired patient, for example, which may include a patient requiring CAPD, protrusions, which can be felt by the user, can be located on the pull ring 14 of the port protector 10, if desired.

A number of thermoplastic materials can be used, such as olefinic, styrenic, or polyamide thermoplastic elastomers or a combination of same, including a minimum of 23% hydrophilic polymeric components. Certain combinations of polyvinyl chloride, plasticizers, and stabilizers can be used.

Preferably, the port protector 10 is constructed from a thermoplastic elastomer composition chosen from the group consisting of: thermoplastic olefinic elastomers; styrenic thermoplastic elastomers; polyamide thermoplastic elastomers; or a combination thereof having at least 23% hydrophilic polymeric components. In a preferred embodiment, the port protector 10 is constructed from a soft polyvinyl chloride material that is injection moldable.

Due to the structure of the port-protector 10 and material used, a container assembly including the port protector 10 that can be located, for example, on either of the ports of the container or a connector extending from a fluid tube, can be terminally sterilized using steam sterilization. A further sterilization step with ionizing radiation is not required. Due to the thickness and material composition of the port protector 10, steam can easily penetrate the top of the port protector 10 sterilizing the interior of a port or connector.

Additionally, the port protector 10 provides the necessary elastomeric properties required by such a product. In this regard, an elastomeric material is chosen that will return substantially to the original shape after having been stretched or maintains a significant retraction force long after the deformation step. In order to maximize processing efficiency, a melt processable rubber, thermoplastic elastomer, is used. The thermoplastic elastomer should provide needed processability. Additionally, the thermoplastic elastomer is chosen so as to provide hydrophilicity and steam permeability.

Referring now to Figure 3, an example of the port protector 10 used on a container assembly 30 is illustrated. Although in this embodiment, the container assembly 30 is a CAPD solution container assembly, of course, any container assembly can be utilized.

In the container assembly 30 of Figure 3, a first, or solution container 32 and a second, or drain container 34 are provided. Fluid lines 36 and 38 extend from the solution and drain containers 32 and 34, respectively. A Y-branch 39 that terminates in a luer connector 40 allows fluid flow from the patient into the drain container 34 and from the solution container 32 into a patient. The connector 40 is, in the illustrated embodiment, a male luer lock connector as is known in the CAPD art.

To protect the connector 40 from touch contamination and other sterility issues, the port protector 10 is positioned thereover. As noted above, due to the structure of the port protector 10, the entire unit 30 can be steam sterilized. Additionally, it is not necessary to position a frangible cannula in the drain line 38.

To determine possible thermoplastic elastomer compositions that can be used, experiments were performed. By way of example, and not limitation, the following examples of materials that could be used are set forth with respect to the below experiments.

To facilitate the selection process of material candidates, screening tests as follows were performed. The material of interest was first molded into flat sheets of about 0.01 inch (250 micron) in thickness. Two sheets of the material approximately 7.5 x 7.5 cm containing 1.0 gram of a desiccant such as calcium chloride or calcium sulfate were heat sealed into a pouch. Upon steam autoclaving at 121°C for 30 minutes, the pouch was cooled to ambient temperatures and the amount of steam that penetrated into the pouch and the resulting weight gain were measured.

For the materials evaluated, the moisture penetration weight gain varied from less than 5% to about 70%. This reflects a wide range of permeabilities to steam under high temperatures and pressures.

### EXAMPLE 1

Santoprene 281-55, a thermoplastic olefinic elastomer by Monsanto was tested. The material showed weight gains of 7 and 8 percent in replica pouch samples. When molded into closure caps, the material exhibited no benefit in microbial kill when tested in a steam autoclave experiment.

### EXAMPLE 2

Kraton G-2705, a styrenic thermoplastic elastomer by Shell was tested in this example. The material showed weight gains of 7.8 ± 1.3%. The material also showed no steam microbial kill in the closure form.

Example 2 demonstrates that Santoprene had very good elastic properties but, when molded alone in the form of a closure cap, was not hydrophilic enough to allow a sufficient amount of steam to permeate the distal end of the cap following the steam sterilization cycle and ensure sterility of the enclosed volume limited by said cap.

### EXAMPLE 3

Pebax-4011, a nylon 6 and tetramethylene glycol based thermoplastic elastomer by Ato Chemie was tested in this example. The material showed good hydrophilicity and logarithmic reduction of microbial spores of 6 when tested as an injection molded closure. However, the molded closure was deemed too rigid for functional purposes.

Example 3 demonstrates that PEBAX 4011 had very good hydrophylicity but, when molded alone in the form of a closure cap, did not have sufficient elastic properties to prove physically functional.

### EXAMPLE 4

A blend of 70% Kraton G-2705 and 30% Pebax-4011, a nylon 6 and tetramethylene glycol based thermoplastic elastomer from ATO Chemie was tested. The material showed a pouch weight gain of 57 and 68% in replica samples, and when injection molded into closures, exhibited a microbial spore logarithmic reduction of 6. In addition, the molded closure possessed sufficiently low rigidity for functionality.

Example 4 demonstrates that a combination of materials identified in Examples 2 and 3 and blended in a proportion including 25% to 35% of PEBAX 4011 would exhibit, when molded in the form of caps, adequate elastic properties and sufficient hydrophilicity to achieve sterility of the enclosed volume limited by said cap.

### EXAMPLE 5

A flexible PVC formulation containing 80 phr of DEHP plasticizer and 7 phr of a epoxidized oil secondary plasticizer/stabilizer along with about 0.2 phr of a standard calcium/zinc stearate heat stabilizer was tested in this example. The material showed a pouch weight gain of about 60%, and when injection molded into cap closures, exhibited a greater than 6 log microbial reduction during steam sterilization. Molded closures from this material were also low in rigidity for functional requirements.

Example 5 demonstrates that PVC according to the formulation described achieves hydrophylicity and physical functionality similar to that of materials mentioned in Example 4. Therefore, materials used in Examples 4 and 5 can successfully be used to mold a sterility protector which is viable both in terms of microbiology (sterility of enclosed volume of sterile closure) and physical functionality.

## Claims

1. A port protector removably attached over an open end of a tubular member (40), the port protector comprising:
a cap portion (12) including a planar closure (18) and side walls (17,19) defining an interior receiving said open end and sealingly engaging the tubular member, the cap portion (12) at least being constructed from a thermoplastic elastomer; a ring (14) extending from the cap portion to facilitate removal of the cap portion (12) from the tubular member, the ring being attached directly to the side walls (17,19) and extending from opposite points on the side walls forming an enclosed opening between the ring and the cap, the ring extending from the end of the side walls opposite to said open end,
characterised in that the closure (18) defines a top of the cap portion (12), extending over said open end and spaced from the ring (14) by the side walls (17,19), the ring (14) extends in a plane in parallel spaced relation to the plane of said closure (18), and the closure (18) has a sufficiently thin cross-sectional thickness to allow steam to pass through the top into the opening of the tubular member during steam sterilization.

2. The port protector of Claim 1 wherein the top (18) of the cap (12) has a thickness (a) of equal to or less than 0.65 millimeters.

3. The port protector of Claim 1 or 2, wherein the entire port protector (10) is constructed from the same material.

4. The port protector of any preceding claim including touch indicators for providing means for identifying products through touch.

5. The port protector of any preceding claim wherein at least portions of the port protector (10) are colour coded to provide visual means for identifying product.

6. The port protector of any preceding Claim wherein the port protector (10) is constructed from at least one material chosen from the group consisting of: thermoplastic olefinic elastomers; styrenic thermoplastic elastomers; polyamide thermoplastic elastomers; and elastomeric compositions having at least 23% by weight hydrophilic polymeric components.

7. A container assembly (30) including:
two containers (32,34), each including an interior, one container (32) being designed to house a solution and a second container (34) for receiving a solution, the first container including a fluid flow line (36) and the second container including a drain line (38), the fluid flow line (36) and drain line (38) being in fluid communication with a connector (40) including a tubular member;
the drain tube (38) not including a frangible cannula; and
a port protector (10) for removably sealing an opening located at an end of the tubular member and constructed according to any preceding claim.

## Patentansprüche

1. Anschlußschutz, der über einem offenen Ende eines rohrförmigen Elements (40) abnehmbar angebracht ist, wobei der Anschlußschutz folgendes aufweist:
einen Kappenbereich (12), der aufweist: einen planaren Verschluß (18) und Seitenwände (17, 19), die einen Innenraum definieren, der das offene Ende aufnimmt und mit dem rohrförmigen Element in abdichtenden Eingriff gelangt, wobei der Kappenbereich (12) wenigstens aus einem thermoplastischen Elastomer gebildet ist; einen Ring (14), der sich von dem Kappenbereich erstreckt, um das Abnehmen des Kappenbereichs (12) von dem rohrförmigen Element zu erleichtern, wobei der Ring unmittelbar an den Seitenwänden (17, 19) angebracht ist und sich von entgegengesetzten Stellen an den Seitenwänden erstreckt unter Bildung einer umschlossenen Öffnung zwischen dem Ring und der Kappe, wobei sich der Ring von dem Ende der Seitenwände erstreckt, das zu dem offenen Ende entgegengesetzt ist,
dadurch gekennzeichnet, daß der Verschluß (18) eine Oberseite des Kappenbereichs (12) definiert, die sich über das offene Ende erstreckt und von dem Ring (14) durch die Seitenwände (17, 19) beabstandet ist, der Ring (14) sich in einer Ebene in paralleler beabstandeter Beziehung zu der Ebene des Verschlusses (18) erstreckt und der Verschluß (18) eine ausreichend dünne Querschnittsdicke hat, um zuzulassen, daß bei Dampfsterilisation Dampf durch die Oberseite in die Öffnung des rohrförmigen Elements eintritt.

2. Anschlußschutz nach Anspruch 1, wobei die Oberseite (18) der Kappe (12) eine Dicke (a) hat, die gleich wie oder weniger als 0,65 mm ist.

3. Anschlußschutz nach Anspruch 1 oder 2, wobei der gesamte Anschlußschutz (10) aus demselben Material gebildet ist.

4. Anschlußschutz nach einem der vorhergehenden Ansprüche, der Berührungsanzeiger aufweist, um Mittel zum Identifizieren von Produkten durch Berührung zu bilden.

5. Anschlußschutz nach einem der vorhergehenden Ansprüche, wobei wenigstens Bereiche des Anschlußschutzes (10) farbcodiert sind, um visuelle Mittel zur Produkterkennung zu bilden.

6. Anschlußschutz nach einem der vorhergehenden Ansprüche, wobei der Anschlußschutz (10) aus wenigstens einem Material gebildet ist, das aus der Gruppe ausgewählt ist, die besteht aus: thermoplastischen olefinischen Elastomeren; thermoplastischen Styrol-Elastomeren; thermoplastischen Polyamid-Elastomeren; und elastomeren Zusammensetzungen, die wenigstens 23 Gew.-% hydrophile polymere Bestandteile haben.

7. Behälteranordnung (30), die folgendes aufweist:
zwei Behälter (32, 34), die jeweils einen Innenraum aufweisen, wobei ein Behälter (32) ausgebildet ist, um eine Lösung zu enthalten, und ein zweiter Behälter (34) ausgebildet ist, um eine Lösung aufzunehmen, wobei der erste Behälter eine Fluiddurchflußleitung (36) und der zweite Behälter eine Ablaufleitung (38) aufweist, wobei die Fluiddurchflußleitung (36) und die Ablaufleitung (38) mit einem Verbinder (40), der ein rohrförmiges Element aufweist, in Fluidverbindung sind;
wobei das Ablaufrohr (38) keine brechbare Kanüle aufweist; und
einen Anschlußschutz (10) zum abnehmbaren dichten Verschließen einer Öffnung, der sich an einem Ende des rohrförmigen Elements befindet und der gemäß einem der vorhergehenden Ansprüche ausgebildet ist.

## Revendications

1. Protecteur d'orifice fixé de façon séparable sur une extrémité ouverte d'un élément tubulaire (40), le protecteur d'orifice comprenant :
un capuchon (12) incluant une paroi de fermeture plane (18) et des parois latérales (17,19) qui définissent un espace intérieur recevant ladite extrémité ouverte et qui sont en contact étanche avec l'élément tubulaire, le capuchon (12) au moins étant fabriqué en un élastomère thermoplastique ; et
un anneau (14) s'étendant à partir du capuchon pour faciliter l'enlèvement du capuchon (12) de l'élément tubulaire, l'anneau étant attaché directement aux parois latérales (17,19) et s'étendant à partir de points opposés sur les parois latérales de manière à définir une ouverture cernée entre l'anneau et le capuchon, l'anneau s'étendant à partir de l'extrémité des parois latérales qui est à l'opposé de ladite extrémité ouverte ;
caractérisé en ce que la paroi de fermeture (18) définit une partie supérieure du capuchon (12), s'étendant sur ladite extrémité ouverte et espacée de l'anneau (14) par les parois latérales (17,19), l'anneau (14) s'étend dans un plan parallèle et espacé par rapport au plan de ladite paroi de fermeture (18), et la paroi de fermeture (18) a une épaisseur en coupe suffisamment mince pour permettre le passage de la vapeur d'eau à travers la paroi supérieure et sa pénétration dans l'ouverture de l'élément tubulaire pendant la stérilisation à la vapeur.

2. Protecteur d'orifice suivant la revendication 1, dans lequel la paroi supérieure (18) du capuchon (12) a une épaisseur (a) égale ou inférieure à 0,65mm.

3. Protecteur d'orifice suivant la revendication 1 ou 2, dans lequel l'ensemble du protecteur d'orifice (10) est fabriqué en la même matière.

4. Protecteur d'orifice suivant une quelconque des revendications précédentes, incluant des indicateurs tactiles pour fournir des moyens d'identification des produits au toucher.

5. Protecteur d'orifice suivant une quelconque des revendications précédentes dans lequel au moins des portions du protecteur d'orifice (10) présentent un codage de couleur pour fournir des moyens visuels d'identification du produit.

6. Protecteur d'orifice suivant une quelconque des revendications précédentes, dans lequel le protecteur d'orifice (10) est fabriqué à partir d'au moins une matière choisie dans le groupe contenant: élastomères thermoplastiques oléfiniques, élastomères thermoplastiques styréniques, élastomères thermoplastiques de type polyamide, et compositions élastomères ayant au moins 23% en poids de composants polymères hydrophiles.

7. Ensemble de récipients (30) comprenant :
deux récipients (32,34) délimitant chacun un espace intérieur, un récipient (32) étant prévu pour contenir une solution et un deuxième récipient (34) étant prévu pour recevoir une solution, le premier récipient comportant une ligne d'écoulement de fluide (36) et le deuxième récipient comportant une ligne de drainage (38), la ligne d'écoulement de fluide (36) et la ligne de drainage (38) étant en communication de fluide avec un connecteur (40) incluant un élément tubulaire ;
le tube de drainage (38) ne comportant pas de canule cassable ; et
un protecteur d'orifice (10) fermant de façon amovible une ouverture située à une extrémité de l'élément tubulaire, ce protecteur étant construit selon une quelconque des revendications précédentes.
